# EUROPEAN PATENT APPLICATION

(11) **EP 1 627 573 A1**
(43) Date of publication of application: **22.02.2006**
(21) Application number: 04104014.8
(22) Date of filing: 20.08.2004
(51) Int. Cl.: A23L 1/22, A23P 1/04, A23G 3/00, A61K 8/11

(54) **A process for the incorporation of a flavor or fragrance ingredient or composition into a carbohydrate matrix**

(71) Applicant: FIRMENICH SA, 1211 Genève 8 (CH)
(72) Inventor: Subramaniam, Anandaraman, East Windsor, New Jersey 08520 (US); McIver, Robert, Clark, Tabernacle, New Jersey 08088 (US); Van Sleeuwen, Rutger, M.T., Somerset, New Jersey 08873 (US)
(74) Representative: Salvaterra-Garcia, Maria de Lurdes

(57) **Abstract**

The invention relates to a hot melt extrusion process for the preparation of a flavor or fragrance delivery system, wherein the quenching of the extruded product to form a glass is carried out with a cooling means of liquid nitrogen.

## Description

### Background of the Invention

The present invention relates to the field of encapsulation. It concerns more particularly the improvement, in terms of cost of processing and safety, as well as of the final product quality, of known processes relating to the incorporation of a volatile ingredient or composition such as a flavor or fragrance compound, into a carbohydrate based matrix. The invention specifically relates to a process for the preparation of a solid flavor or fragrance particulate composition comprising the preparation of an aqueous solution of carbohydrate material, the evaporation of water from said solution, the emulsification of a flavor or fragrant ingredient or composition in the obtained concentrated solution, the cooling of the mixture, its extrusion into a cold medium to vitrify the carbohydrate/active material melt and finally the drying of the particulate composition thus obtained. The process of the invention is characterized in that the cooling step takes places by contacting the melt with a specifically selected cooling means, more particularly liquid nitrogen or a spray thereof, or yet a metal surface cooled with liquid nitrogen.

Encapsulation techniques are widely used, in particular in the flavor and fragrance industries, to alleviate problems caused by the volatility and lability of perfume and flavor active ingredients. In fact, due to the nature of the latter, losses of volatile components might occur during storage or processing, prior to incorporation of these active ingredients in a final consumer product. Moreover, encapsulation of active ingredients is also used to ensure their proper and controlled release from a matrix system.

It is not surprising therefore to observe that, in order to reduce or eliminate the stability or release problems associated with volatile and labile flavor or fragrance components, many attempts have been made to encapsulate such ingredients in carbohydrate matrices, so as to reduce their volatility or lability. This results in the preparation of stable free flowing powders containing flavor or fragrance ingredients or compositions for subsequent flavor or fragrance release when the particles thus obtained are incorporated into a final consumer product or when such a product is eventually consumed.

The prior art has therefore developed a number of techniques for producing, in particular in the flavor industry, solid essential oil particulate compositions. Amongst these techniques, extrusion methods typically rely on the use of carbohydrate materials constituting the matrix, which are heated to a molten state and combined with essential oils or flavor ingredients, before being extruded and finally quenched to form a glass which protects the flavor. Typical products obtained by such methods and used in the flavor and fragrance industries are dry granular delivery systems wherein the active ingredients are uniformly distributed as droplets throughout a carbohydrate glass.

One of the earliest examples of a process of pertinence to the field of the invention is US patent no. 3,041,180 to Swisher, which describes a process of the type above-mentioned, wherein the quenching, melt vitrification step is carried out by extruding the hot emulsion into a cold organic solvent, the temperature of which may vary from room temperature to as low as -18 C.

Another significant example of the prior art disclosure in this field is US 3,704,137 which describes an essential oil composition formed by mixing an oil with an antioxidant, separately mixing water, sucrose and hydrolysed cereal solids with DE below 20, emulsifying the two mixtures together, extruding the resulting mixture in the form of rods into a relatively cool liquid solvent, removing the excess solvent and finally adding an anti-caking agent. The solvent exemplified is isopropanol (IPA).

Subsequent patents relating to similar processes are US 4,610,890 and US 4,707,367 which describe a process for preparing a solid essential oil composition having a high content of essential oil, which composition is prepared by forming an aqueous solution containing sugar, a starch hydrolysate and an emulsifier. The essential oil is blended with the aqueous solution in a closed vessel under controlled pressure to form a homogeneous melt, which is again extruded into a relatively cold organic solvent, dried and combined with an anti-caking agent. Again, the cold organic solvent cited is IPA.

The above-mentioned patents, and others which are referenced in such documents, are merely illustrative of the considerable volume of patent literature related to the fixation of flavor or fragrance ingredients in various encapsulation matrices, in particular by way of hot melt extrusion processes, and in essence these documents all disclose encapsulation processes which resort to the use of a cooling liquid to quench the extruded melt, the temperature of this cooling material being possibly as low as -20 C. In a vast majority of the cases, the selected organic liquid is IPA.

Typically, this cooling organic solvent performs two critical functions in the manufacture of the encapsulate, i. e. the rapid cooling of the extruded strands to form a dense carbohydrate glass enclosing the active fragrance or flavor active material and the washing of any residual flavor or fragrance oil from the surface of the cooled/quenched strands. Both these functions are key to obtaining a stable extruded product.

IPA is by far the most currently used cooling means in such processes. The use of IPA in such extrusion processes has however some drawbacks mostly related to safety and environmental issues. This is a flammable material with a flash point of 11 C and its vapor is classified as volatile organic compound (VOC), such that spent IPA is considered a hazardous waste requiring specialized equipment for its handling and storage. Moreover, it has been observed that some IPA is occasionally encapsulated in the extruded material and cannot be completely removed by the final drying step.

In view of these prior art documents, there is a clear need for an improved method for producing flavor or fragrance-containing capsules or particles, wherein the safety and efficiency of the method is enhanced and likewise the quality of the product obtained, without significantly changing the latter's essential characteristics such as moisture, Tg and flavor content.

### Brief Summary of the Invention

Now, surprisingly, we have been able to overcome the drawbacks encountered in the prior art methods by providing a process for the encapsulation of an active ingredient, further referred to as an "active", namely a flavor or fragrance ingredient or composition, comprising the steps of :
a) combining and blending a flavor or fragrance ingredient or composition with a matrix comprising an aqueous solution of at least a carbohydrate material and optionally an emulsifier, under temperature and stirring conditions useful to produce a uniform melt thereof having a moisture content below 10%;
b) extruding the uniform melt through a die ;
c) chopping, cutting, grinding or pulverising the material obtained as it exits the die or after cooling the melt ; and
d) optionally drying;

wherein the cooling of the melt in step c) is carried by contacting the extruded material with a liquid nitrogen or a metal surface cooled by liquid nitrogen.

According to a preferred embodiment of the process of the invention, the cooling of the extruded melt takes place by extruding into a liquid nitrogen bath.

We have been able to establish that, despite the very low temperature of liquid nitrogen (-196 C), and unlike what could have been expected at such low temperatures, no cracking or creation of fissures in the particles was observed. Moreover the physical properties of the particulate material, and its content in encapsulated fragrance or flavor oil, were similar to those obtained with the prior art methods which used cold organic solvents at much higher temperatures. Likewise as regards the surface oil levels on the particles. In other words, no adverse effects of such low temperatures were observed and this was a totally unexpected result.

On the other hand, the invention provides a process which is safer and allows a more rapid and efficient glass formation than prior known methods of this type. Liquid nitrogen is non-flammable, non-toxic and natural. Separation of the particles and disposal of spent liquid nitrogen takes place by evaporation to the air. The process of the invention thus allows elimination of the IPA bath which is typical of the current processes, and a simplification of the equipment currently used in the final stages for removal of most of the IPA and IPA handling equipment such as is necessary for the chilling, capturing such a VOC and disposing thereof. This applies to all known such manufacturing methods, whether batch or continuous processes and, as a result, such processes according to the invention are more cost effective.

The prior art above cited is totally silent as regards the possible use of cooling/quenching materials at temperatures below -20 C, as in fact one would have expected that extremely low temperatures would have caused the extruded strands to shatter or at least to have shown different surface morphology when compared to the extruded products obtained with for example IPA cooling. Yet, we have established that the use of liquid nitrogen provides all the advantages associated with the use of a liquid as the quenching means in hot melt type extrusion processes, such as for example the optimal contact of the liquid with the extruded strands which allows dense placement of the holes in the extrusion die, whilst avoiding the drawbacks associated with prior known processes which use organic solvents and more particularly IPA. The latter include the need for mechanical means to separate the particles from the bulk cooling solvent and recycling of the latter for repeated use, whilst liquid nitrogen evaporates in air and does not require recovery for environmental reasons. Moreover, we have observed that there is no residual encapsulated fluid in the particles when liquid nitrogen is used. The latter also allows the manufacture of particulate flavor and fragrance compositions which cannot be manufactured with current processes because of the solubility of the carbohydrate matrices in organic solvents, namely IPA, or because, at the extrusion point in time, their Tg (glass transition temperature) is less than the IPA cooling temperature.

### Detailed Description of the Invention

The present invention will now be described in greater detail.

The invention concerns a process for the preparation of a solid flavor or fragrance particulate composition as cited above. According to an embodiment of the process, the cooling step is carried out via extrusion of the melt into a liquid nitrogen bath.

The process above-described embraces a variety of extrusion techniques, depending notably on the materials used and on the amount of water added in the first step of the process, which may have to be reduced during a drying step in order to obtain a final product having an acceptable glass transition temperature (Tg). In fact, the critical glass transition temperature may be at least above 20°C and preferably above 40°C for the major part of applications. However in some cases, it may be useful to prepare delivery systems which have glass transition temperatures below ambient temperature as disclosed in WO 96/11589, the content of which is hereby included by reference. The proportions in which water is employed in the present invention therefore vary in a wide range of values which the skilled person is capable of adjusting and choosing as a function of the carbohydrate glass used in the matrix and the required Tg of the final product.

Such a process may be carried out batch-wise or in a continuous mode.

According to a particular batch type embodiment, and with the exception of the means for cooling and quenching the melt into a glass encapsulating the active materials, the typical conditions for this process are similar to those of processes for encapsulating flavors, as described for example in US patents Nos. 4,610,890 and 4,707,367, the contents of which are hereby included by reference. The products obtained by this type of methods are based on the formation of a melt and extrusion of the latter at an appropriate temperature. Typically, the material or composition is combined and blended with an aqueous mixture of a sugar, a starch hydrolysate and preferably an emulsifier, and this aqueous mixture is then heated to the boiling point of water or a temperature slightly above, but preferably not above 130°C, to form a homogeneous melt, which is then extruded through a die. The molten mass which exits the die can then either be chopped as it is still in a plastic state (melt granulation or wet granulation techniques) before being cooled, or be directly cooled to form the extruded solid, the shape and size of which can be adjusted as a function of the extrusion parameters before being grinded. According to the invention, the chopped extrudate or the molten strands as they exit the die plunge into liquid nitrogen, or into a container cooled by a liquid nitrogen bath, to be quenched and form an amorphous glass encapsulating the flavor or fragrance ingredient or composition. The thus cooled strands or particles are then collected without the need for any mechanical separation equipment.

The final step of the process is the drying stage, the aim of which is to reduce the moisture content of the extruded product to the desired level. Once the particles have been dried, by means of an anticaking agent such as silicon oxide for example, they are mixed with a free flow agent and sifted to meet size specification.

The quenching step by means of liquid nitrogen cooling is used advantageously with both prior known batch processes, such as those described in detail in the above-mentioned US patents, and continuous processes. In particular, it can be advantageously applied to the continuous process which is the object of International patent application PCT/IB2004/000880, filed March 10, 2004 and claiming a priority of March 19, 2003, the contents of which are hereby included by reference.

The latter describes in detail a process characterized by the fact that it is entirely carried out in a continuous manner and comprises, in the continuous layout of the process, two heat exchangers providing for, on the one hand, the evaporation of water during the concentration of the aqueous solution of carbohydrate material in step a), and on the other hand the cooling of the mixture of carbohydrate and active ingredient before extrusion. The presence of the first heat exchanger allows an accurate concentration of the aqueous carbohydrate solution while maintaining the mean residence time of the solution in the heat exchanger to a minimum, so as to reduce the damages on carbohydrate constituents of the matrix. On the other hand, the second heat exchanger provides an accurate way to cool down the mixture of carbohydrate and active ingredient to the desired extrusion temperature, thus providing at the end of the process a product which is more uniform in terms of flavor or fragrance retention and in particular as regards the retention of volatile materials. Furthermore, an accurate control of the extrusion temperature allows to better control the size distribution of the particulate composition finally obtained.

Thus, according to this particular embodiment of the present invention which relates to a hot melt type extrusion method carried out continuously, the process for the preparation of a solid flavor or fragrance particulate composition comprises the steps mentioned before, all steps being carried out continuously and the homogeneous melt formed in step a) being obtained by means of two heat exchangers appropriately placed in the lay-out of the continuous process path for this step. Firstly, the aqueous solution of carbohydrate material is concentrated via the first heat exchanger to reduce the amount of water in the syrup prior to being admixed with the flavor or fragrance material to be encapsulated, and, following the admixture of the latter, the syrup/active material mixture is then passed onto the surface of the second heat exchanger to bring its temperature to the desired extrusion value. Steps b) and c) of the process remain unchanged and the quenching of the extruded material is carried out via liquid nitrogen cooling as described previously.

A continuous process means a computer controlled process, by opposition to a batch process, wherein all operations are manual. Essentially, the different processing steps of this embodiment of the process of the invention are each carried out by different pieces of equipment which, when appropriately sized and connected together, are combined to make a continuous process. The latter allows to accurately control the process variables, in particular the extrusion temperature, and therefore to provide a final product of consistent quality. Moreover, compared to the batch process embodiment, it allows to lower the cost of manufacture of the final product, for larger volumes produced. Practically, while the batch conditions disclosed in the process from the prior art, such as that above, allow to encapsulate at most about 75 to 85% by weight of the quantity of oil combined and emulsified in the matrix, the continuous process of the present invention allows to effectively encapsulate more than 90% of the active ingredient combined with the concentrated candy.

The heat exchangers used in the evaporation and cooling operations of step a) are heat exchangers with, respectively swept and scraped surfaces.

A swept or scraped surface heat exchanger is basically made up of a cylinder with a finished inner surface, a rotor mounted approximately on the cylinder axis, and pins or other means carried by the rotor for mounting scraping or sweeping blades to continuously sweep or scrape depending on the direction of rotation of the rotor, layers of heated or cooled liquid from the inner cylinder wall, the heating or cooling being effected usually by a hot or cold medium in an annulus jacket surrounding the heat exchange cylinder. Such type of swept or scraped surface heat exchanger is disclosed for instance in US 3,633,664. Optionally, there may be a passage for a second heat exchanger to heat or cool the cylindrical outer surface of the rotor, as disclosed in US 4,073,339, so that the product mass passing through the chamber in the jacketed cylinder can be heated or cooled from both the outside and the inside of the mass, simultaneously with the mixing of the mass by the action of scraper means.

Many other models of swept or scraped surface heat exchangers are described in the literature, for instance in US 3,955,617 or yet in US 5,518,067 wherein the apparatus disclosed is particularly adapted to the heating or cooling of fluids having a certain viscosity. The present invention is not limited to one particular type of heat exchanger. Many types of apparatus commercially available suit the purpose of the continuous process of the present invention. Therefore, a more detailed description of the apparatus is not needed here, as this is well described in the literature and well known by a skilled person in the art.

The first heat exchanger is preferably a swept surface heat exchanger, and allows to evaporate water from the initial aqueous solution of carbohydrate material or syrup, to form a concentrated carbohydrate solution or candy. The presence of such a heat exchanger provides an efficient way to reduce the mean exposure residence time of the syrup, respectively candy, to heat. This further improves on the batch process in that it advantageously reduces the possible damages due to heat over the carbohydrate constituent of the matrix. As a result, in the particular case where sucrose is present among the carbohydrate materials, risks of browning the carbohydrates and off-flavor development are minimized. Moreover, in another particular case where the matrix would comprise gum materials, excess heat exposure may have a detrimental effect on emulsifying properties of the latter materials. Here again, such a drawback is minimized thanks to the continuous process of the present invention.

On the other hand, the presence of the first above-mentioned heat exchanger in the continuous process of the invention implies that the rate of feed of the syrup to the heat exchanger for evaporation is controlled, and as a consequence, the concentrated formed candy is uniform in color and flavor or fragrance. This is also an improvement upon the batch processes, where the variability in the evaporation stage sometimes results in visual and flavor (from caramelization of the carbohydrates) differences between individual batches.

The second heat exchanger used within the framework of the present invention allows to cool down to an accurate and defined temperature in the fourth step of the process, the candy-active mixture (emulsion) which is going to be extruded. More particularly, a skilled person in the art is aware that, in all hot melt type extrusion processes, for a given combination of matrix materials, moisture and flavor or fragrance ingredient or composition, there is an optimum extrusion temperature. In fact, if the latter is too high, it may induce a de-mixing of the emulsion, resulting in low volatile content in the finished product, as well as degradation of low boiling components which could thus flash off as they exit the holes of the die. Besides, such temperature conditions may also provide a low active content in the final product, an alteration of the flavor or fragrance profile and a weakening of the strands of extruded product by an expansion of the strands exiting the die. On the other hand, if the extrusion temperature is too low, the mixture will be difficult to extrude due to high viscosity and will thus require higher pressure conditions to be extruded, which can lead to a poor control of the diameter of the strands exiting the die. This low temperature also possibly results in long extrusion times which adversely impact throughput rates.

Now, in an extrusion batch process, the temperature of extrusion cannot be as well-controlled. In fact, although the temperature of the jacket of a vessel is set, it cannot strongly influence the final temperature of the material prior to extrusion. This is improved in the continuous process of the invention, however, where, after the mixture exits the emulsifying unit, the emulsion is passed through a heat exchanger, preferably a scraped surface heat exchanger, to cool the mixture to an accurate desired extrusion temperature. As a result, the product formed at the end of the process is even more uniform in retention of active ingredient, in particular as regards the more volatile components. Besides, a better control of the extrusion temperature, allows to produce a particulate composition with a narrow size distribution, or in other words, to improve the control of the particle size.

Other aspects and advantages of the process of the invention are disclosed in the detailed description contained in the above-cited International patent application.

The first step of all embodiments of the process according to the invention is the preparation of an aqueous solution of at least one carbohydrate material, which solution is termed "syrup". As the carbohydrate material, there can be used any carbohydrate or carbohydrate derivative which can be processed through extrusion techniques to form a dry extruded solid. Particular examples of suitable materials include those selected from the group consisting of sucrose, glucose, lactose, levulose, fructose, maltose, ribose, dextrose, isomalt, sorbitol, mannitol, xylitol, lactitol, maltitol, pentatol, arabinose, pentose, xylose, galactose, hydrogenated starch hydrolysates, maltodextrin, agar, carrageenan, other gums, polydextrose, synthetic polymers such as polyvinyl alcohol, semi-synthetic polymers such as succinylated starch, cellulose ethers, proteins such as gelatin, and derivatives and mixtures thereof.

According to a particular embodiment of the invention, there will be used maltodextrin or mixtures of maltodextrin with at least one material selected from the group consisting of sucrose, glucose, lactose, levulose, maltose, fructose, isomalt, sorbitol, mannitol, xylitol, lactitol, maltitol and hydrogenated starch hydrolysates, preferably a maltodextrin having a dextrose equivalent not above twenty (≤20) and more preferably a DE of 18.

The above-mentioned carbohydrate materials are hereby given by way of example and it is not to be interpreted as limiting the invention. Although polysaccharides are mentioned above as specific examples, it is clear that any material which is extrudable and currently used as a matrix material in the production of extruded solids appropriate for flavor or fragrance applications, or which proposed in the future as a suitable material for extrusion type manufactured products is adequate for the aim of the invention and is therefore hereby included in the latter.

The aqueous solution or syrup prepared in the first step of the invention typically comprises from 12 to 40% by weight of water relatively to the total weight of the solution, preferably from 18 to 25% by weight.

To prepare the syrup, a portion of the water is metered into a mixing tank. The carbohydrate material is batch added or conveyed to the dry solid weight tank until the desired quantity is reached. This material is dropped into the mixing tank containing water. A second portion of water is sprayed onto the top of the carbohydrate in the tank to aid in wetting and dispersion. This process is repeated when the matrix comprises more than one carbohydrate component. Also into this mixing tank, may be added other matrix ingredients such as pH modifiers, emulsifyers, gums or colorants, as required. For instance, suitable emulsifiers include sulfoacetates of mono- and diglycerides as well as polyglycerol esters, lecithin and modified lecithin. These emulsifiers are given by way of example but they are not to be interpreted as limiting the invention, as any emulsifier having a hydrophobic part and a hydrophilic part can be used within the framework of the invention. The contents of the mixing tank are mixed with high shear to form a homogeneous dispersion. The dispersion is further transferred by gravity to a heating tank located on the bottom. The dispersion is extracted or continuously pumped from the tank through the multitude heat exchanger and back to the hot tank in a loop. The syrup is heated to about 60 to 80°C to form a solution, preferably to 70 to 80°C. Once the syrup is heated to the desired temperature, it is transferred to a holding tank for concentration and further processing.

The water is then evaporated from the syrup to form a concentrated carbohydrate solution. The moisture content of the latter after concentration varies according to the matrix composition and to the active ingredient quantity to be incorporated. Typically, the moisture of the candy varies between 2 and 11% by weight and preferably between 3.5 and 7% by weight relative of the total weigh of the concentrated solution.

In practice, in the continuous process of the invention, the mixture is pumped from the holding tank through a swept surface heat exchanger in which the water is evaporated to achieved the desired moisture level in the candy. Typically, the temperature of the heat exchanger is comprised between 105 and 150°C. In a particular embodiment, it is comprised between 115 and 135°. This temperature will be set as a function of the matrix composition. The contents of the heat exchanger are discharged into a tank in which the evaporated water is vented to the atmosphere and the concentrated candy falls to the bottom of the tank.

The flavor or fragrance ingredient or composition referred to as "active" is dispersed throughout the candy to form the mixture to be extruded. The terms flavor or fragrance ingredient or composition as used in the context of the processes of the invention are deemed to define a variety of flavor and fragrance materials of both natural and synthetic origin. They include single compounds and mixtures. The process of the invention may be employed to manufacture encapsulated volatile and labile components which may be in liquid or solid form, hydrophilic or hydrophobic. Specific examples of such components may be found in the current literature, e.g. in Perfume and Flavor Chemicals by S. Arctander, Montclair N.J. (USA) ; Fenaroli's Handbook of flavour Ingredients, CRC Press or Synthetic Food Adjuncts by M.B. Jacobs, van Nostrand Co., Inc. and are well-known to the person skilled in the art of perfuming, flavoring and/or aromatising consumer products, i.e. of imparting an odor or taste to a consumer product.

Natural extracts can also be encapsulated by the process of the invention. These include e.g. citrus extracts, such as lemon, orange, lime, grapefruit or mandarin oils, or coffee, tea, cocoa, mint, vanilla or essential oils of herbs and spices, amongst other.

It is well known that, for an effective encapsulation of an active ingredient in a delivery system, this active ingredient must be uniformly dispersed as small droplets throughout the matrix materials. Advantageously, and according to the continuous process of the invention, the concentrated mixture obtained in the precedent stage of the process exits the tank at a fixed rate and is pumped through an in-line high shear homogenizer. Immediately before this homogenizing unit, the active ingredient is metered into the processing line at a fixed rate. Residence time in the homogenizing unit is less that 10 seconds.

Again, compared with a batch process, the continuous embodiment of the process allows to shorten the exposure time of the mixture to high shear conditions. Since the action of high shear blades within a viscous matrix results in a significant amount of heat generation due to friction, this continuous embodiment helps prevent damage of heat labile components. Moreover, the uniform rate of addition of active ingredient to a constant flow of candy of uniform moisture content, results in a more consistent finished product, compared to individual batches of the batch process of the invention.

In the continuous embodiment, the second heat exchanger, through which surface the carbohydrate and flavor/fragrance emulsion is passed, is preferably a scraped surface heat exchanger to cool the mixture to a temperature optimized between the limits explained above. Typically, the extrusion temperature is comprised between 102 and 135°C, preferably between 112 and 130°C. The extrusion temperature is here optimized as a function of the matrix compositions, and composition and level of flavor or fragrance material. A skilled person in the art is capable of defining this optimized temperature. The heat exchanger then allows to accurately reach this temperature in particular, and through this control, to provide an advantageously uniform product in terms of retention of active ingredient, as well as a better control of the final particulate composition size.

In both the batch and continuous processes the extrusion step consists in forcing the molten mixture through the holes of a die, thus forming the strands of product which fall into the liquid nitrogen bath. As pointed out before, the control of the extrusion step is important for product quality and yield. Again, according to the continuous embodiment of the invention, a pump may be supplied which operates at a fixed speed resulting in a constant extrusion rate. Typical continuous extrusion pressures are, within the framework of the invention, in the range of 1 to 3x10⁵ Pa. Therefore, thanks to these low extrusion pressures, expansion of strand diameter at the exit of the die holes is minimized relative to the batch process and a better uniformity of size can be obtained. Besides, de-mixing of phases is better avoided. The total heat exposure time is uniform for all material produced and finally, the uniform extrusion rate advantageously results in a more uniform strand diameter than it is possible with the batch process.

The extruded strands fall into the liquid nitrogen cooling means an this provides for very rapid cooling to transform the molten strands into an amorphous glass which is then dried as described above.

In the continuous process according to the invention, the particles are preferably dried and cooled in either a multiple tray type dryer or a fluid bed dryer with typical residence times of 2 hours or 45 minutes respectively. These dryers are given by way of example, as any known other type of continuous dryer also works fine.

As previously mentioned the processes of the invention make it possible to obtain extruded products which contain no encapsulated cooling fluid, i.e. liquid nitrogen.

The quenching step by means of liquid nitrogen cooling can be generally applied to any prior extrusion process of the hot melt type which involves vitrification of extruded strands into a quenching medium aimed at forming an amorphous glass encapsulating flavor or fragrance materials in particular. Thus it can be generally applied in the context of all the prior art methods described in the patents cited throughout this introduction and it is also convenient within the context of the processes described in more recent patent literature, of which WO 01/74178, US 5,709,895, WO 02/65858 and US 6,607,778 are very pertinent examples. In as much as all such known processes can advantageously comprise a quenching step wherein liquid nitrogen is used to replace the cooling organic solvent generally described therein, and such replacement is easily carried out by the skilled person in the manner described in this instant patent application, the scope of the present includes all such varied manners of carrying out steps a), b) and c) and the contents of such prior art documents relating to those steps, modified to incorporate the teachings herein as regards the quenching of the molten extrudate, are hereby included by reference.

The extruded solids of the invention, obtained according to the processes laid out above, are particularly appropriate for the delivery of hydrophilic or hydrophobic flavoring or perfuming ingredients contained therein. These extruded solids are typically granulated products which are stable against moisture and oxygen and prevent degradation of the perfuming or flavor ingredient or composition encapsulated therein. This is a result of the fact that the latter is homogeneously and uniformly distributed in the amorphous extruded matrix and perfectly incorporated within. These granulated products are far easier to handle, as they produce no significant amounts of dust when processed into the foods, powder beverages, chewing-gums, pharmaceuticals, toothpastes and other edibles and consumer products into which they are incorporated. If used to perfume consumer products, they will be typically incorporated is solid or creamy products, in particular, soaps, cosmetics and powder detergents. In general, any consumer product in which extruded particulate compositions are currently used to impart or modify the taste or odor thereof are an object of the present invention. The latter also includes the particulate flavor and fragrance compositions obtained via the processes described herein.

### Short Description of the Drawings

Figures 1 and 2 show photographs, taken under the conditions described in Example 3, of the products obtained via the process of the invention involving extrusion into IPA, respectively liquid nitrogen.
Figures 3 and 4 show scanning electron microscopy (SEM) images of the products obtained via the process of the invention involving extrusion into IPA, respectively liquid nitrogen.

### Detailed Description of Specific Embodiments of the Invention

The invention will be now described in a more detailed manner by way of examples, wherein the temperatures are indicated in degrees Celsius, and the abbreviations have the usual meaning in the art.

### Example 1

### Batch process according to the invention

An extruded product was manufactured with the following ingredients, in the proportions indicated, using a batch type process.

| Ingredient | grams | % dry |
|---|---|---|
| Maltodextrin 18 DE | 9660 | 46.26 |
| Sucrose | 8920 | 42.72 |
| Orange oil | 2100 | 10.06 |
| Lecithin | 200 | 0.96 |
| Water | 5220 | ----- |
| | 26100 | 100.00 |

The maltodextrin and sucrose were dissolved in water and heated to 130°C to reduce the water content to approximately 6%. The lecithin was dissolved in the orange oil and then mixed with agitation to form a uniform melt. The mixture was extruded through a die plate with 0.8 mm holes under 3 bar pressure into a basket with 0.5 mm perforations that was immersed in a receiving vessel containing approximately 30 liters of liquid nitrogen. Once the extrusion was completed, the cold strands collected in the basket were removed from the liquid nitrogen bath and placed in a dryer. After drying, 1% silicon dioxide was added as a free flow agent. The final product contained 9.8% flavor by weight, 4.3% moisture and had a glass transition temperature of 46°C.

### Example 2

### Continuous process according to the invention

A syrup solution of the following composition :

| Ingredients | Parts by weight |
|---|---|
| Sucrose | 40 |
| Maltodextrin 18DE | 40 |
| Water | 20 |

was pumped at 80° into a first heat exchanger, at a rate of 8.0 kg/min.

Steam (approximately at 150°) was supplied to the jacket of the heat exchanger to evaporate water from the syrup. Steam temperature and flow rate were regulated to give the desired moisture content after evaporation. Residence time in the heat exchanger was 2 min.

The concentrated syrup plus water exited the first heat exchanger into a tank were the water vapor was removed.

A pump removed the melt from the tank and a flavor oil (mixture of 96 parts cold pressed orange oil, 4 parts lecithin) was injected into the processing line at a rate of 1.5 kg/min.

The mixture of melt and flavor oil passed for 10 s through an in-line high shear mixer to form an emulsion.

The emulsion passed through a second heat exchanger to cool to a temperature of 120° as measured at the exit of the heat exchanger. The temperature of the medium (hot water) flowing through the jacket of the heat exchanger was regulated to control the exit temperature of the emulsion. The product then passed through the extrusion die, into liquid nitrogen. The particles there-obtained were dried in a fluid bed dryer with a residence time of 45 min.

The particles obtained contained 16.8% by weight of orange oil and 4% by weight of moisture, relative to the total weight of a particle, and 95% of the initially combined cold pressed oil, was found to be in an encapsulated form at the end of the process.

### Example 3

### Comparative example

Two 20 kg pilot batches of carbohydrate matrices similar to that described in example 1, each containing 10% orange oil, were similarly extruded according to the batch method into liquid nitrogen, respectively isopropyl alcohol (IPA), followed by drying. The process followed was similar to that described in example 1 above.

The finished products were compared - they appeared the same and had no odor of orange oil. Analysis of the final products showed no difference between the two methods of extrusion cooling with respect to moisture content, glass transition temperature or flavor content. There were no discernible differences of surface morphology between the samples obtained with the two cooling treatments when examined by macro photography or by scanning electron microscopy (SEM).

Examination by macro photography was done to see if there were visual differences in the finish products of the same matrix in terms of surface gloss, striations (from the extrusion) and cracking. In order to examine the fine surface detail, the particles were examined by using SEM. Of particular interest was whether the extremely low temperature of the liquid nitrogen resulted in fissures or cracks of the particles. Macro photography enabled large numbers of particles to be examined and a general idea of the typical appearance for each product to be determined. The SEM, although allowing examination of the surface in much greater detail, was restricted to single particles.

The conditions under which the products were examined were the following.

Glass transition temperature (Tg) : was determined with a Perkin-Elmer DSC 7. Samples (about 10 mg each) were cooled to -20°C and held for 5 minutes. Temperature was ramped at 10°C/min to 120°C followed by quenching at -20°C. After a 5-minute hold, the temperature was ramped to 120°C at a rate of 10°C/minute. Tg was determined by the inflection of the heat flow curve of the rescan. Duplicate samples of each product were run.

Particle photographs : were taken with a Canon EOS IDS 35mm digital camera body (11 mPixel) with a Canon EF MP-E 65mm 1-5X macro lens. Sample was illuminated by a Macro Twin Lite MT-24EX flash unit.

Scanning electron microscopy (SEM) : Samples were attached on SEM stubs with double sided tape and coated for approximately 3 minutes with Gold-Palladium under Argon gas using a Balzars SCD 020 Sputter-coater. The SEM instrument for these images was a JEOL JSM 35C Electron Microscope set at 15kV. The magnifications listed are valid for the original images, which have been resized for inclusion in this report. The marker in the lower portion of each image can be used for scaling.

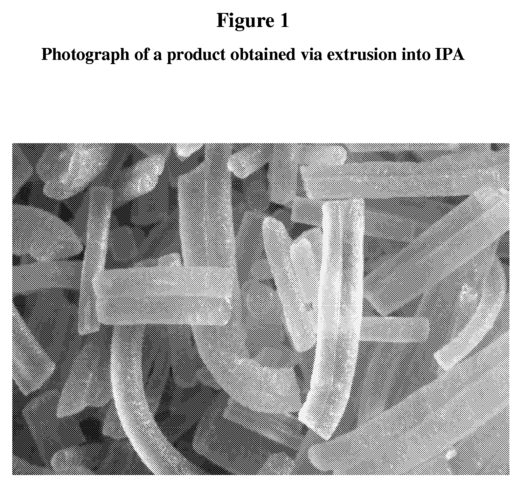

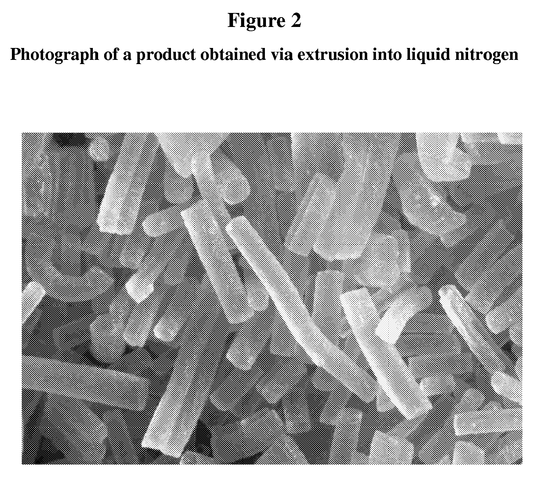

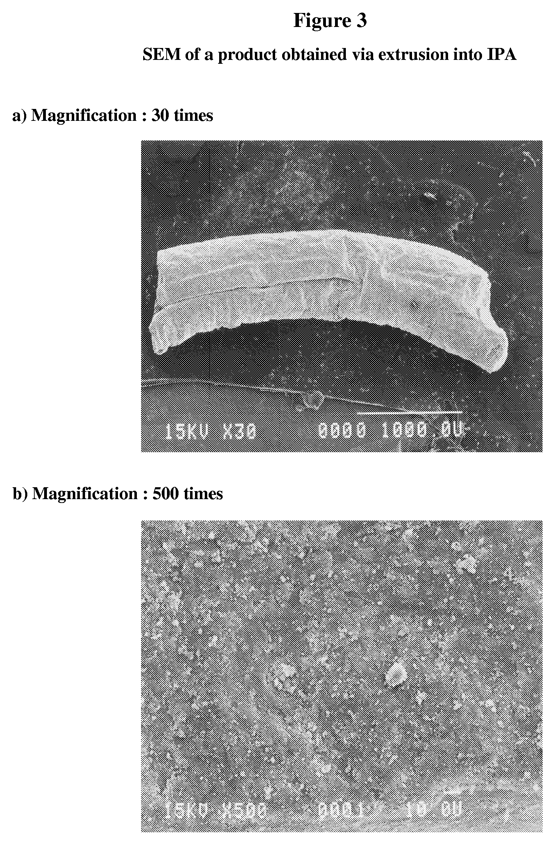

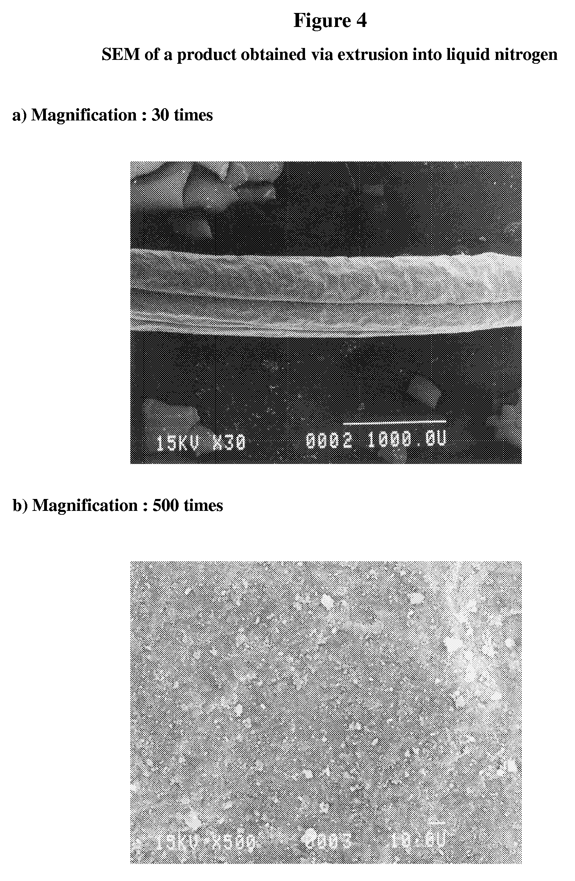

The visual examination of the products extruded into either IPA or liquid nitrogen by macro photography, presented in Figures 1 and 2, showed no discernible differences. Similarly, by SEM the products appeared indistinguishable. The surface wrinkles seen at low magnification in Figures 3 and 4 were present in both samples and detailed surface structure appeared similar in the two samples at the higher magnification.

The similarity of the two products is also apparent from the analytical data presented hereafter :

**Table 1 : Analytical data for products extruded into IPA or liquid Nitrogen**

| **Cooling medium** | **Moisture %, KF** | **Tg °C** | **Aw 25 °C** | **Flavor % w/w** |
|---|---|---|---|---|
| IPA | 4.5 | 38 | 0.23 | 9.0 |
| liq N₂ | 4.4 | 38 | 0.25 | 9.2 |

The Tg values for the two products were typical at the measured moisture contents. The lack of orange odor from the products indicated that residual surface orange oil was low for both products.

Similar tests were carried out with a large variety of other samples having quite a wide range of matrix materials. The two cooling methods were always compared and the results obtained were always consistent, i.e. the trials trials showed that extruding into liquid nitrogen produced particles with similar moisture, Tg and flavor content as those of products made by extrusion into IPA.

The extreme low temperature of liquid nitrogen (-196 °C) did not induce cracking or fissures in the extruded particles compared to IPA extruded material when examined by macro photography or SEM. No significant differences between the treatments were observed in the particle's morphology.

These experiments indicated that extrusion into liquid nitrogen neither damaged the physical structure of the particles nor altered their properties, unlike what would have been expected.

## Claims

1. A process for the encapsulation of a flavor or fragrance ingredient or composition, comprising the steps of :
a) combining and blending a flavor or fragrance ingredient or composition with a matrix comprising an aqueous solution of at least a carbohydrate material and optionally an emulsifier, under temperature and stirring conditions useful to produce a uniform melt thereof having a moisture content below 12% by weight;
b) extruding the uniform melt through a die ;
c) chopping, cutting, grinding or pulverising the material obtained as it exits the die or after cooling the melt ; and
d) optionally drying;
wherein the cooling of the melt in step c) is carried by contacting the extruded material with a cooling medium of liquid nitrogen.

2. A process according to claim 1, wherein the extrusion temperature is comprised between 90 and 130° and the molten uniform mixture is chopped as it exits the die to provide a product having a glass transition temperature Tg which is essentially the same as that of the matrix carrier material.

3. A process according to claim 1, wherein the matrix consists of a hot carbohydrate melt of 3 to 12% moisture.

4. A process according to claim 1, wherein all the steps are carried out continuously and the homogeneous melt formed in step a) is obtained by means of two heat exchangers appropriately placed in the lay-out of step a) of the continuous process.

5. A process according to claim 4, wherein an aqueous solution of carbohydrate material is concentrated via a first heat exchanger to reduce the amount of water in the syrup prior to being admixed with the flavor or fragrance material to be encapsulated, and, following the admixture of the latter, the syrup/active material mixture is passed onto the surface of a second heat exchanger to bring the temperature of said mixture to be extruded to the desired extrusion value.

6. A process according to claim 5, wherein the first heat exchanger is a swept surface heat exchanger.

7. A process according to claim 5, wherein second heat exchanger is a scraped surface heat exchanger.

8. A process according to claim 5, wherein the syrup is heated to a temperature comprised between 105 and 150 °C at the first heat exchanger.

9. A process according to claim 5, wherein the mean residence time of the syrup in the first heat exchanger is comprised between 1 and 10 min.

10. A process according to claim 4, wherein the aqueous solution of step a) contains from 12 to 40% by weight of water relative to the total weight of the solution.

11. A process according to claim 4, wherein the aqueous solution of step a) is prepared by means of conveying the starting materials from a dry solid weight tank to a mixing tank and a heating tank, and then pumping from the heating tank through a multi-tube heat exchanger and back to the hot tank in a loop.

12. A process according to claim 5, wherein the mixture at the end of concentrating step at the first heat exchanger has a moisture content comprised between 2 and 11% by weight.

13. A process according to claim 5, wherein the admixture of the fragrance or flavor active material is carried out by means of a high shear homogenizer in which the residence time of the mixture is of less than 1 min.

14. A process according to claim 5, wherein the mixture is cooled to a temperature comprised between 102 and 135°C before extrusion.

15. A process according to claim 5, wherein the extrusion step is carried out at a pressure comprised between 1x10⁵ Pa and 6x10⁵ Pa.

16. An extruded system for the delivery of a fragrance or flavor active material susceptible of being obtained by a process according to claim 1.

17. An extruded system for the delivery of a fragrance or flavor active material susceptible of being obtained by a process according to claim 5.

18. A delivery system according to claim 17, wherein comprising a matrix of sugar or a sugar derivative.

19. A delivery system according to claim 18, wherein the matrix is selected from the group consisting of sucrose, glucose, lactose, levulose, fructose, maltose, ribose, dextrose, isomalt, sorbitol, mannitol, xylitol, lactitol, maltitol, pentatol, arabinose, pentose, xylose, galactose, hydrogenated starch hydrolysates, maltodextrin, Stabilite ® agar, carrageenan, gums, polydextrose and derivatives and mixtures thereof.

20. A delivery system according to claim 18, wherein the matrix is formed of maltodextrin or of a mixture of maltodextrin and at least one material selected from the group consisting of sucrose, glucose, lactose, levulose, maltose, fructose, isomalt, sorbitol, mannitol, xylitol, lactitol, maltitol and hydrogenated corn syrup.

21. A delivery system according to claim 18, wherein the matrix includes maltodextrin, preferably having a DE equivalent lower than 20.

22. Flavored product or composition which comprises as an active ingredient, a delivery system according to claim 16 or 17.

23. Flavored product or composition according to claim 22, in the form of a chewing gum or a soft chewy confection, an ice-cream, a biscuit, a pharmaceutical preparation or a tooth-paste.

24. Perfumed product or composition which comprises as an active ingredient, a delivery system according to claim 16 or 17.

25. Perfumed product or composition according to claim 24, in the form of a soap, a cosmetic preparation, a deodorant or a solid detergent or cleaning product.
